(19) Europäisches Patentamt
European Patent Office
Office européen des brevets

(11) Veröffentlichungsnummer : **0 014 981**
**B1**

(12) **EUROPÄISCHE PATENTSCHRIFT**

(45) Veröffentlichungstag der Patentschrift : **24.02.82**

(51) Int. Cl.³ : **A 61 K 31/53**// C07D405/14

(21) Anmeldenummer : **80100806.1**

(22) Anmeldetag : **18.02.80**

# (54) Cytostatisch wirkende Arzneimittel bzw. pharmazeutische Zubereitungen.

(30) Priorität : **24.02.79 DE 2907349**

(43) Veröffentlichungstag der Anmeldung : **03.09.80 (Patentblatt 80/18)**

(45) Bekanntmachung des Hinweises auf die Patenterteilung : **24.02.82 Patentblatt 82/08**

(84) Benannte Vertragsstaaten : **AT BE CH FR GB IT LU NL SE**

(56) Entgegenhaltungen : **Keine !**

(73) Patentinhaber : **Henkel Kommanditgesellschaft auf Aktien**
**-Patentabteilung- Postfach 1100 Henkelstrasse 67**
**D-4000 Düsseldorf 1 (DE)**

(72) Erfinder : **Budnowski, Manfred, Dr.**
**Am Nettchesfeld 24**
**D-4000 Düsseldorf 13 (DE)**
Erfinder : **Schnegelberger, Harald, Dr.**
**Stieglitzweg 2**
**D-5653 Leichlingen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 014 981 B1

Imprimerie Jouve, 18, rue St-Denis, 75001 Paris, France

Cytostatisch wirkende Arzneimittel bzw. pharmazeutische Zubereitungen

Es ist bekannt, daß eine Reihe von alkylierenden Substanzen eine cytostatische beziehungsweise cytotoxische Wirkung entfalten. Die bekanntesten Verbindungen leiten sich vom sogenannten Stickstofflost ab. Darüber hinaus ist es auch bekannt, wenigstens zwei Epoxidgruppen im Molekül enthaltende Verbindungen als Cancerostatika zu verwenden. Derartige Verbindungen sind beispielsweise das 4,4'-Bis(2,3-epoxypropyl)-di-piperidinyl(1,1') und das 1,2-15,16-Diepoxy-4,7,10,13-tetraoxohexadecan. Allerdings haben die letzteren Verbindungen keine wesentliche Verbesserung in der cytostatischen Behandlung gebracht und werden kaum verwendet. Lediglich zur Behandlung von Hirntumoren werden sie noch gelegentlich eingesetzt. Einer breiteren Anwendung steht auch die begrenzte Löslichkeit der erwähnten Verbindungen entgegen.

Aufgabe der vorliegenden Erfindung war es, leichtlösliche, insbesondere wasserlösliche Verbindungen mit cytostatischer beziehungsweise cytotoxischer Wirkung zu finden, die gegen möglichst viele Malignome und Leukämieformen wirksam sind. Sie sollten bei wenigstens einer der folgenden tierexperimentell erzeugbaren Leukämien L 1 210 und P 388 beziehungsweise Melanom B16 beziehungsweise LL-Carcinom gemäß den Vorschriften des « Cancer Chemotherapy Reports » (Sept. 1972) ein T/C-Verhältnis (siehe Seite 47) von mindestens 150 % haben.

Gelöst werden kann diese Aufgabe durch Verbindungen mit cytostatischer Wirksamkeit gemäß der allgemeinen Formel

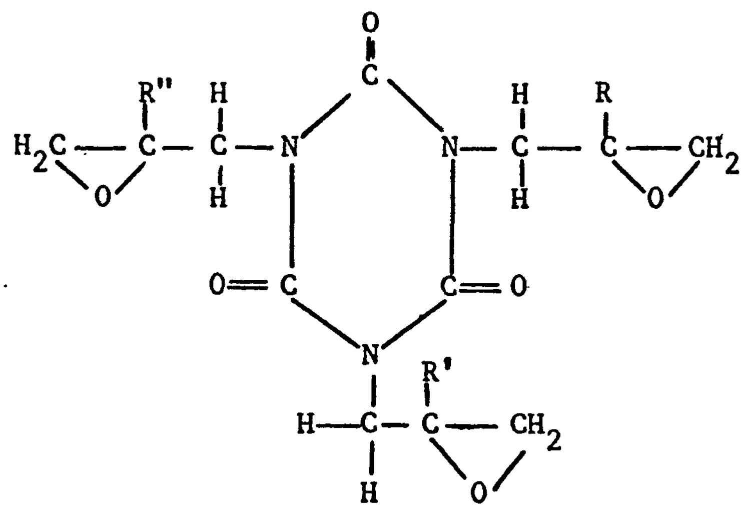

in der R, R' beziehungsweise R" entweder gleiche oder verschiedene niedere Alkylreste mit 1 bis 4 Kohlenstoffatomen und/oder Wasserstoff sind. Bevorzugt sind solche Verbindungen, bei denen R, R' beziehungsweise R" entweder Wasserstoff und/oder eine Methylgruppe sind. Die Verbindungen sollen also verwendet werden als cytostatische Wirksubstanzen zur Therapie von malignen Neoplasien. Diese Mittel kommen gegebenenfalls zusammen mit üblichen pharmazeutischen Hilfsstoffen und/oder Trägersubstanzen zur Anwendung.

Bei diesen einen Isocyanuratring enthaltenden Glycidylverbindungen handelt es sich um prinzipiell bekannte Substanzen. Diese wurden bereits früher in mehr oder weniger reiner Form synthetisiert und zur Herstellung von vernetzten Kunststoffen verwendet. Obwohl die bevorzugten Verbindungen, in denen R, R' beziehungsweise R" Wasserstoff ist, sich durch eine für diesen Verbindungstyp erstaunlich gute Löslichkeit in Wasser beziehungsweise hydrophilen, wassermischbaren Lösungsmitteln auszeichnen, ist bisher an eine Verwendung in wasserhaltigen beziehungsweise wäßrigen, hydrophilen, insbesondere für pharmazeutische Zwecke oder in Arzneimitteln noch nicht gedacht worden.

Wenn die drei Substituenten R, R' und R" gleich sind, fallen zwei Substanzen an, die zueinander diastereomer sind. Das gleiche gilt, wenn kein Alkylrest vorhanden ist, sondern der Substituent Wasserstoff ist. Man bezeichnet diese Verbindungen dann als α- oder β-Triglycidylisocyanurat (vergl. Angew. Chemie (1968) S. 851/2). Ihre Herstellung ist in der deutschen Patentschrift 12 11 650 sowie der US-Patentschrift 3,337,509 beschrieben. Diese beiden Verbindungen sind leicht zugänglich durch Umsetzung von Cyanursäure mit Epichlorhydrin im Überschuß und Abspaltung von HCl unter Bildung des Oxiranringes. Aus dem Rohprodukt kann man sie durch fraktionierende Kristallisation, beispielsweise in Methanol, Methylenchlorid, Glycolmonomethylether, Glycolmonoethylether oder dergleichen gewinnen. Die sogenannte α-Form zeigt in reiner Form einen Schmelzpunkt von 106 °C, während die β-Form einen Schmelzpunkt von 158 °C aufweist. Obwohl für technische Verwendung im allgemeinen eine Trennung nicht erforderlich ist, wurde im vorliegenden Fall die Wirksamkeit der beiden Isomeren getrennt untersucht. Aufgrund der unterschiedlichen Löslichkeit in Wasser, gegebenenfalls im Gemisch

mit den vorher erwähnten Lösungsmitteln, ist eine saubere Trennung ohne weiteres möglich. Reine Produkte zeigen einen Epoxidsauerstoffgehalt, der zwischen 16 und 16,2 Gewichtsprozent liegt. In an sich bekannter Weise kann man natürlich auch aus dem diastomeren α-beziehungsweise β-Triglycidylisocyanurat die Enantiomeren in mehr oder minder reiner Form gewinnen und einsetzen, wobei sich gegebenenfalls die Wirksamkeit noch steigern läßt.

Zur Verwendung als Cancerostatika sollten die Wirksubstanzen mittels geeigneter Vehikel appliziert werden. Im vorliegenden Fall hat sich die Verwendung von Wasser, gegebenenfalls zusammen mit verträglichen Glycolethern, wie Glycolmonoethylether oder Butylenglycolmethylether oder Propylenglycolmethylether bewährt, insbesondere wenn der Wirkstoff parenteral appliziert werden soll. Bei oraler Verabreichung sind die pharmazeutisch üblichen Hilfs- beziehungsweise Trägerstoffe anwendbar, sofern sie eine entsprechende Verträglichkeit mit den Glycidylverbindungen aufweisen.

Im Tierexperiment hat sich die Verwendung von frisch hergestellten, wäßrigen Lösungen, die i.p. gegeben werden, als zweckmäßig erwiesen. Von α-beziehungsweise β-Triglycidylisocyanurat kann bei Mäusen die Maximaldosis p. die 100 mg/kg Maus betragen. Deutliche toxische Wirkungen treten erst bei höherer Dosierung auf. Als optimale Dosierung hat sich in vielen Fällen eine Gabe von 30 mg bis 60 mg/kg Maus während einer Applikationsdauer von 1 bis 9 Tagen bewährt.

Die genannten Verbindungen sind wirksam gegen verschiedene Leukämieformen sowie maligne Neoplasmen, wie Lungencarcinom, Coloncarcinom, Melanome, Ependymoblastom und Sarcome. Es hat sich gezeigt, daß in einigen Fällen eine deutliche Überlegenheit gegen Cyclophosphamid und Fluoruracil festzustellen ist.

Selbstverständlich ist es möglich, nicht nur das α- und β-Triglycidylisocyanurat als Cancerostatika einzusetzen, sondern auch die anderen genannten Verbindungen, in denen R beziehungsweise R' beziehungsweise R'', zumindest zum Teil eine Methylgruppe darstellt. Die therapeutische Wirksamkeit korreliert bei den angegebenen Verbindungen u.a. mit der Löslichkeit. Dies ist zu beachten, wenn die Alkylgruppen gemäß Definition für R bzw. R' bzw. R'' mehr als ein Kohlenstoffatom enthalten.

Die Herstellung des erfindungsgemäßen Arzneimittels zur in Verbindung mit anderen allgemein üblichen alkylierenden Substanzen, wie Derivaten des Stickstofflosts oder auch Fluoruracil ist zur Behandlung der vorstehend genanniten Neoplasmen selbstverständlich möglich.

## Beispiele

Die nachfolgenden Beispiele wurden durchgeführt nach Testvorschriften des National Cancer Institute Bethesda, Maryland 200 014, veröffentlicht in « Cancer Chemotherapy Reports » Part. 3, Sept. '72, Vol. 3, Nr. 2. Als Wirksubstanz wurde verwendet α-Triglycidylisocyanurat (F 106 °C) sowie β-Triglycidylisocyanurat (F 158 °C), beide mit 16,1 % Epoxidsauerstoffgehalt (Herstellung siehe US-PS 3,337,509).

Im Beispiel 9 wurde Tri-(2-methylglycidyl)-isocyanurat verwendet (DE-OS 19 54 531, Ciba). Das Tri-(2-methylglycidyl)-isocyanurat wurde aus Kaliumcyanat und Methallylchlorid sowie nachfolgender Epoxidation der Triallylverbindung hergestellt.

In einem Autoklaven wurden 86 g Kaliumcyanat, 95 g Methallylchlorid sowie 391 g Acetonitril gemischt und unter Stickstoffatmosphäre auf 150 °C während 3 Stunden erhitzt. Nach dem Abkühlen wurde ausgefallenes Salz (KCl) abfiltriert und das Acetonitril im Rotationsverdampfer abdestilliert. Das angefallene feste Rohprodukt wurde mit Cyclohexan extrahiert. Beim Eindampfen kristallisierte das Tri-(2-methylallyl)-isocyanurat aus. Schmelzpunkt : 84 bis 85 °C, Jodzahl : 264,0 (berechnet 261,3).

20 g des bevorstehend beschriebenen hergestellten Tri-(2-methylallyl)-isocyanurats wurden in 300 ml Methylenchlorid gegeben und anschließend mit 15,85 g m-Chlor-perbenzoesäure versetzt. Das Gemisch ließ man 70 Stunden im Kühlschrank bei 4 °C stehen. Dann wurde die ausgefallene 3-Chlorbenzoesäure abfiltriert. Das Filtrat wurde mit 10 %iger Sodalösung und anschließend mit Wasser gewaschen. Die Trocknung der Methylenchloridlösung erfolgte mit wasserfreiem Natriumsulfat. Nachdem keine Persäure mehr festgestellt worden war, wurde das Methylenchlorid im Rotationsvakuumverdampfer abdestilliert. Der Rückstand wurde in Ethylether aufgenommen und daraus umkristallisiert. Schmelzpunkt : 69 bis 74 °C.

Es wurden 12,9 g erhalten. Dies entspricht einer Ausbeute von 55,7 % der Theorie.

Der Epoxidgehalt betrug 13,7 % (berechnet 14,1 %). Im IR-Spektrum waren die typischen Isocyanurat-Banden vorhanden bei 1 700 $cm^{-1}$ und 1 455 $cm^{-1}$. Das $^1$H-NMR in $CDCl_3$ zeigt bei δ1,4 ppm die Protonen der $CH_3$-Gruppe am C-Atom-2 der Glycidylgruppe. Die Protonen am C-Atom-1 zeigen ein AB-System bei 4,1 ppm, die am C-Atom-3 ein AB-System mit kleinerer Kopplungskonstante bei 2,6 ppm. Die Struktur ist ferner durch $^{13}$C-NMR und Massenspektrum gesichert.

Alle wäßrigen 1 %igen Injektionslösungen wurden unmittelbar vor der Applikation frisch hergestellt.

## Beispiel 1

Bei Mäusen wurde gemäß Protokoll 1 200 (Seite 9 l.c.) die Tumorart P 388 (Leucämie) i.p. mit $10^6$ Zellen/Maus gesetzt. Die unbehandelten Tiere hatten eine mittlere Überlebensdauer von 10,5 Tagen.

Nach 9-tägiger i.p. Behandlung mit 100 mg/kg α-TGI p. die wurde die mittlere Lebenserwartung

gegenüber der Kontrollgruppe auf 285 % (T/C, Verlängerungsrate) beobachtet. Die Hälfte der behandelten Mäuse lebte länger als 40 Tage und ist als geheilt anzusehen.

Wurden nur 50 mg/kg p. die während 9 Tagen appliziert, betrugen die entsprechenden Werte 200 % T/C und 17 % Heilung. Die entsprechenden Werte bei β-TGI waren für 100 mg/kg p. die 228 % T/C und für 50 mg/kg p. die 179 % T/C. Bei jeweils 17 % der Versuchstiere wurde Heilung beobachtet.

Vergleichsversuch

Eine gegenüber Cyclophosphamid (NSC 26271) resistente Leukämieform von P 388 wurde mit α-TGI und Cyclophosphamid behandelt. Jede Gruppe bestand aus 10 Mäusen.

Bei Gaben von 40 mg/kg α-TGI während 1 bis 9 Tagen überlebten alle Mäuse 60 Tage (T/C 478 %). Demgegenüber überlebten die mit 180 mg/kg Cyclophosphamid behandelten Mäuse keine 30 Tage (T/C 150 %).

Beispiel 2

Es wurde Beispiel 1 wiederholt mit 9-maligen Dosen von 25 mg/kg p. die und folgende Beobachtungen gemacht:

α-TGI ergab 196 % T/C und β-TGI 174 % T/C.

Beispiel 3

Bei Mäusen wurde gemäß Protokoll 1.100 (Seite 7 l.c.) Leukämie L 1 210 durch i.p. Verabreichung von 0,1 ml verdünnter Erregerlösung entsprechend $10^5$ Zellen erzeugt. Bei der Kontrollgruppe betrug die mittlere Überlebensdauer 8 Tage (m.s.t.).

A. Eine Gruppe (6 Mäuse) erhielt vom 1. bis 9. Tag 50 mg/kg p. die an α-TGI i.p. verabfolgt. Die Überlebensdauer stieg auf 23,8 Tage entsprechend einem T/C von 297 %. 30 Tage erreichten 3 Mäuse, das heißt, es wurde eine Heilungsrate von etwa 50 % erreicht.

Eine andere Gruppe erhielt vom 1. bis 9. Tag 50 bzw. 100 mg/kg an β-TGI p. die i.p. verabfolgt. Die mittlere Überlebensdauer betrug 16,3 bzw. 25,2 Tage entsprechend einem T/C von 203 % bzw. 315 %.

B. Der Einfluß des Behandlungsplans auf die therapeutische Wirksamkeit von α-TGI bei Tumor L 1 210 nach 30 Tagen ist der nachfolgenden Tabelle I zu entnehmen.

Tabelle I
Abhängigkeit der Wirksamkeit von der Behandlungsplanung bei α-TGI

| Dosis p. die in mg/kg | Überlebende/ganze Gruppe | |
|---|---|---|
| | leukäm. M. | Kontrollgruppe |
| 50 mg 5x i.p. (1. bis 5. Tag) | 5/10 | 7/8 |
| 40 mg 9x i.p. (1. bis 9. Tag) | 8/10 | 8/8 |

Beispiel 4

Bei Mäusen wurde gemäß Anweisung 1.300 das 1/10 Homogenat Melanom B 16 jeweils 0,5 ml pro Maus i.p. verabfolgt (vergl. S. 11 l.c.).

Die Kontrollgruppe hatte eine mittlere Überlebenszeit von 15,8 Tagen (m.s.t.).

Behandelte Gruppen erhielten vom 1. bis 9. Tag verschiedene Mengen α-TGI i.p. In der nachfolgenden Tabelle II ist die mittlere Überlebensdauer und T/C in Abhängigkeit der p. die gegebenen Menge Wirkstoff wiedergegeben.

Tabelle II
Wirkung von α-TGI in Abhängigkeit von der Dosis

| mg α-TGI | m. s. t. (Tage) | T/C |
|---|---|---|
| 50 | 37,0 | 234 % |
| 25 | 36,0 | 227 % |
| 12,5 | 29,8 | 188 % |

Beispiel 5

Gemäß Protokoll 1.400 (Seite 13 l.c.) wurden bei Mäusen Zellen s.c. eines 1 $mm^3$ Stücks Lungencarcinom (Lewis) implantiert. Dann erfolgte vom 1. bis 11. Tag i.p. Verabfolgung von 40 mg/kg p. die an α-TGI bzw. 90 mg/kg p.die an β-TGI.

Die Hemmung an Metastasen betrug bei α-TGI 92 % und bei β-TGI 87 % gegenüber der Kontrollgruppe nach 23 Tagen.

Beispiel 6

Bei einer Testgruppe von Mäusen wurde durch intracerebral 1 $mm^3$ eines Gewebes von Ependymoblastom implantiert. Die mittlere Überlebensdauer bei der Kontrollgruppe betrug 19,3 Tage.

Bei einer Behandlung mit α-TGI von 40 mg/kg p. die 9 Tage lang konnte ein T/C von 165 % erreicht werden.

Beispiel 7

Durch i.p.-Verabfolgung von $10^6$ Zellen pro Maus wurde das Sarcom 180 gesetzt. Die unbehandelte Kontrollgruppe hatte eine mittlere Überlebenszeit von 20,2 Tagen.

Zur Behandlung wurden 30 mg/kg pro Maus an α-TGI von 1 bis 9 Tagen i.p. verabfolgt. Es ergab sich ein T/C von 183 %.

Beispiel 8

A. Gruppen von 10 Mäusen wurde etwa 1 $mm^3$ Coloncarcinom 38 s.c. implantiert. Das mittlere Tumorgewicht betrug bei der unbehandelten Kontrollgruppe nach 20 Tagen : 512 mg/Maus.

Eine Gruppe erhielt am 2. und 9. Tag 50 mg/kg p. die an α-TGI und eine weitere Gruppe zusätzlich am 16. Tag die gleiche Menge. Die mittleren Tumorgewichte betrugen 153 mg beziehungsweise 183 mg pro Maus.

B. Durch i.p. Implantation (1 $mm^3$ Coloncarcinom 26) wurde bei Gruppen von jeweils 10 Mäusen ein Ca gesetzt. Die Behandlung erfolgte durch i.p.-Verabfolgung von α-TGI. In der nachfolgenden Tabelle III sind die Ergebnisse in Abhängigkeit der jeweils am 1., 5. und 9. Tag applizierten Menge wiedergegeben.

Tabelle III
Überlebenschancen in Abhängigkeit von der Behandlungsplanung

| mg/kg | Ergebnis |
|---|---|
| 25 mg i.p. | 25 Tage alle Mäuse lebend<br>60 Tage 6 von 10 Mäusen lebend |
| 50 mg i.p. | 60 Tage noch 10 Mäuse lebend |
| 100 mg i.p. | 49 Tage noch alle Mäuse lebend<br>60 Tage noch 4 Mäuse lebend |

Der Positivvergleich durch Behandlung mit Fluoruracil ergab, daß von 10 Mäusen am 60. Tag nur noch 2 lebend waren (mittlere Überlebensdauer 55 Tage).

Beispiel 9

Bei Mäusen wurde gemäß Protokoll 1 200 (Seite 9 l.c.) die Tumorart P 388 Leucämie i.p. mit $10^6$ Zellen/Maus gesetzt. Die unbehandelten Tiere hatten eine mittlere Überlebensdauer von 10 Tagen.

Nach 9-tägiger i.p. Behandlung mit 100 mg/kg Tri-(2-methylglycidyl)-isocyanurat p.die wurde die mittlere Lebenserwartung gegenüber der Kontrollgruppe auf 170 % (T/C, Verlängerungsrate) beobachtet.

Wurden nur 50 mg/kg p. die während 9 Tagen appliziert, betrug der entsprechende Wert 150 % T/C.

**Ansprüche**

1. Arzneimittel mit cytostatischer Wirksamkeit der allgemeinen Formel

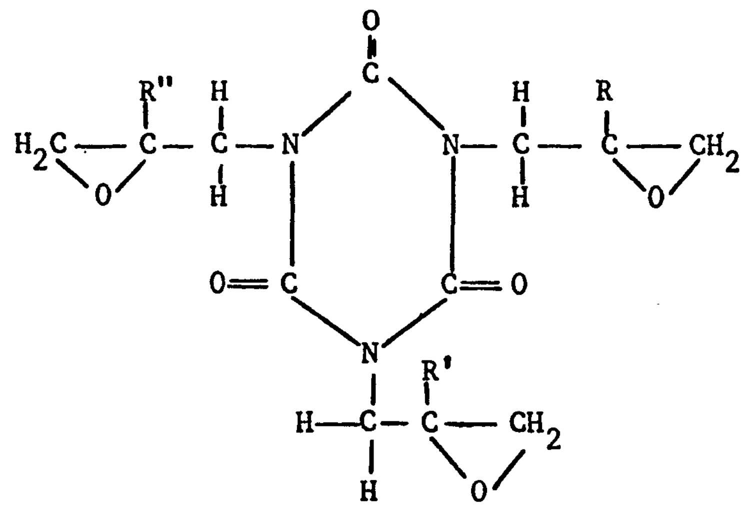

in der R, R' beziehungsweise R" entweder gleiche oder verschiedene niedere Alkylreste mit 1 bis 4 Kohlenstoffatomen und/oder Wasserstoff sind zur Therapie maligner Neoplasien.

2. Arzneimittel nach Anspruch 1, wobei R, R' beziehungsweise R" entweder Wasserstoff und/oder eine Methylgruppe sind und insbesondere R wie R' wie R" Wasserstoff sind.

3. Arzneimittel nach Ansprüche 1 und 2, wobei Wirksubstanz α-und/oder β-Triglycidylisocyanurat und/oder eine der optisch aktiven Formen des Triglycidylisocyanurats ist.

4. Verbindungen gemäß Ansprüche 1 bis 3 zur Verwendung bei der Therapie maligner Neoplasien gegebenenfalls zusammen mit üblichen pharmazeutischen Hilfsstoffen und/oder Trägersubstanzen.

**Claims**

1. Cytostatically active medicaments corresponding to the following general formula

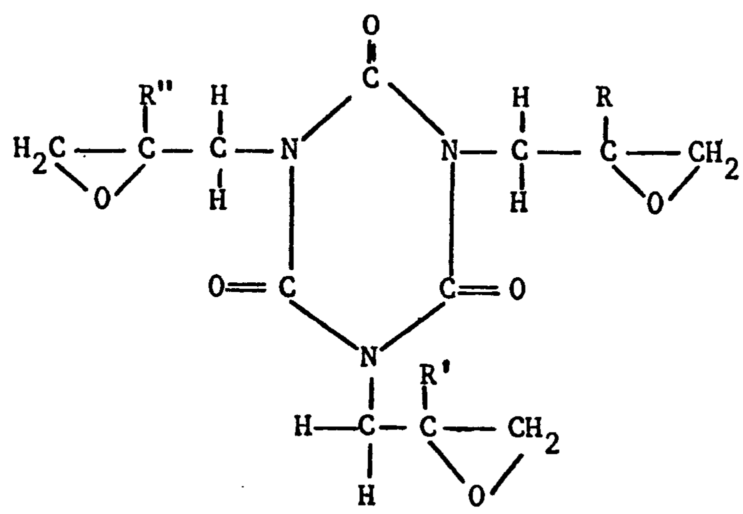

in which R, R' and R" are the same or different and represent lower alkyl radicals containing from 1 to 4 carbon atoms and/or hydrogen, for the treatment of malignant neoplasms.

2. Medicaments as claimed in Claim 1 in wich R, R' and R" represent either hydrogen and/or a methyl group, more particularly hydrogen.

3. Medicaments as claimed in Claims 1 and 2 in which the active principle is α- and/or β-triglycidyl isocyanurate and/or one of the optically active forms of triglycidyl isocyanurate.

4. Compounds as claimed in Claims 1 to 3 for use in the treatment of malignant neoplasms, optionally in conjunction with standard pharmaceutical auxiliaries and/or vehicles.

**Revendications**

1. Médicament à activité cytostatique de formule générale

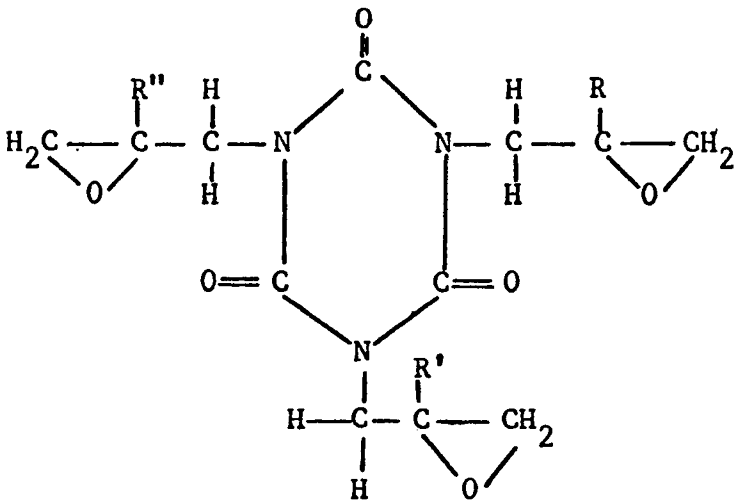

dans laquelle R, R' et R" sont des radicaux alcoyle inférieurs identiques ou différents ayant 1 à 4 atomes de carbone et/ou de l'hydrogène, pour la thérapie des néoplasies malignes.

2. Médicament selon la revendication 1, caractérisé en ce que R, R' et R" sont de l'hydrogène et/ou un groupe méthyle et en particulier R comme R' et R" sont de l'hydrogène.

3. Médicament selon les revendications 1 et 2, caractérisé en ce que la substance active est de l'isocyanurate de triglycidyle α et/ou β et/ou une des formes optiquement actives d'isocyanurate de triglycidyle.

4. Composés selon les revendications 1 à 3 pour l'utilisation dans la thérapie de néoplasies malignes éventuellement conjointement avec les substances auxiliaires et/ou substances de support pharmaceutiques usuelles.